# EUROPEAN PATENT APPLICATION

(11) **EP 2 251 831 A2**
(43) Date of publication of application: **17.11.2010**
(21) Application number: 10160999.8
(22) Date of filing: 26.04.2010
(51) Int. Cl.: G06T 15/00

(54) **Ultrasound system and method for rendering volume data**

(30) Priority: 11.05.2009 KR 20090040626
(71) Applicant: MEDISON CO., LTD., Kangwon-do 250-870 (KR)
(72) Inventor: Lee, Suk Jin, 135-851, Seoul (KR); Kim, Sung Yoon, 135-851, Seoul (KR)
(74) Representative: Schmid, Wolfgang

(57) **Abstract**

There is disclosed an embodiment for volume data rendering. A first processor forms volume data by using a plurality of ultrasound data. A user interface coupled to the first processor receives region of interest (ROI) setting information including a size and a position of the ROI. A second processor coupled to the first processor sets a plurality of sampling start points along edges of the volume data, a plurality of sampling points and a ray-casting direction on the volume data based on the ROI setting information. The GPU is configured to move sampling start points positioned at an inside or outside of the ROI to be positioned at the ROI to render the volume data.

## Description

### CROSS-REFERENCE TO RELATED APPLICATIONS

The present application claims priority from Korean Patent Application No. 10-2009-0040626 filed on May 11, 2009, the entire subject matter of which is incorporated herein by reference.

### TECHNICAL FIELD

The present invention generally relates to ultrasound systems, and more particularly to volume rendering of volume data within a region of interest by using a graphic processing unit (GPU) in an ultrasound system.

### BACKGROUND

The ultrasound system has become an important and popular diagnostic tool due to its non-invasive and non-destructive nature. Modem high-performance ultrasound imaging diagnostic systems and techniques are commonly used to produce two or three-dimensional images of internal features of patients (target objects).

Generally, the ultrasound system may provide a three-dimensional ultrasound image including clinical information such as spatial information and anatomical figures of the target objects, which cannot be provided by a two-dimensional ultrasound image.

The ultrasound system may form volume data by transmitting and receiving ultrasound signals to and from a target object. The ultrasound system may include a central processing unit (CPU) for rendering volume data to form three-dimensional ultrasound images. Volume rendering may be performed by a significant number of data operations, which may increase the occupation of CPU resources. Thus, a heavy overload may be imposed upon the CPU. To resolve this problem, a graphic processing unit (GPU), which is a relatively high speed graphic chipset, has been recently employed for the volume rendering.

Conventionally, to perform the volume rendering upon volume data within a region of interest using ray casting, the GPU may cast a virtual ray from a view plane to the volume data. Accordingly, there is a disadvantage in that artifacts may be produced due to rendering an unnecessary region, and thus, the data operations of the rendering may be increased.

### SUMMARY

An embodiment of the present invention for volume data rendering is disclosed herein. In one embodiment, by way of non-limiting example, an ultrasound system for volume data rendering, comprises: a first processor configured to form volume data by using a plurality of ultrasound data; an user interface coupled to the first processor and being configured to receive region of interest (ROI) setting information including a size and a position of the ROI; and a second processor coupled to the first processor and being configured to set a plurality of sampling start points along edges of the volume data as well as a plurality of sampling points and a ray-casting direction on the volume data based on the ROI setting information, the GPU being further configured to move sampling start points positioned at an inside or outside of the ROI to be positioned at the ROI to render the volume data.

In another embodiment of the present invention, a method for volume data rendering, comprises: a) forming a volume data by using a plurality of ultrasound data; b) receiving region of interest (ROI) setting information including a size and a position of the ROI; c) setting the ROI on the volume data based on the ROI setting information; d) setting a plurality of sampling start points along edges of the volume data, a plurality of sampling points and a ray-casting direction on the volume data based on the ROI setting information; and e) moving sampling start points positioned at an inside or outside of the ROI to be positioned at the ROI to render the volume data.

In yet another embodiment of the present invention, a computer readable medium comprising instructions that, when executed by a processor performs a volume data rendering method of an ultrasound system, cause the processor to perform steps comprising: a) forming a volume data by using a plurality of ultrasound data; b) receiving region of interest (ROI) setting information including a size and a position of the ROI; c) setting the ROI on the volume data based on the ROI setting information; d) setting a plurality of sampling start points along edges of the volume data, a plurality of sampling points and a ray-casting direction on the volume data based on the ROI setting information; and e) moving sampling start points positioned at an inside or outside of the ROI to be positioned at the ROI to render the volume data.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- FIG. 1: is a block diagram showing an illustrative embodiment of an ultrasound system.
- FIG. 2: is a block diagram showing an illustrative embodiment of an ultrasound data acquisition unit.
- FIG. 3: is a block diagram showing an illustrative embodiment of a graphic processing unit.
- FIG. 4: is a schematic diagram showing an illustrative embodiment of volume data and a region of interest.
- FIG. 5: is a schematic diagram showing an illustrative embodiment of sampling start points, sampling points and a ray-casting direction.
- FIGS. 6 and 7: are schematic diagrams showing illustrative embodiments of setting sampling start points.

### DETAILED DESCRIPTION

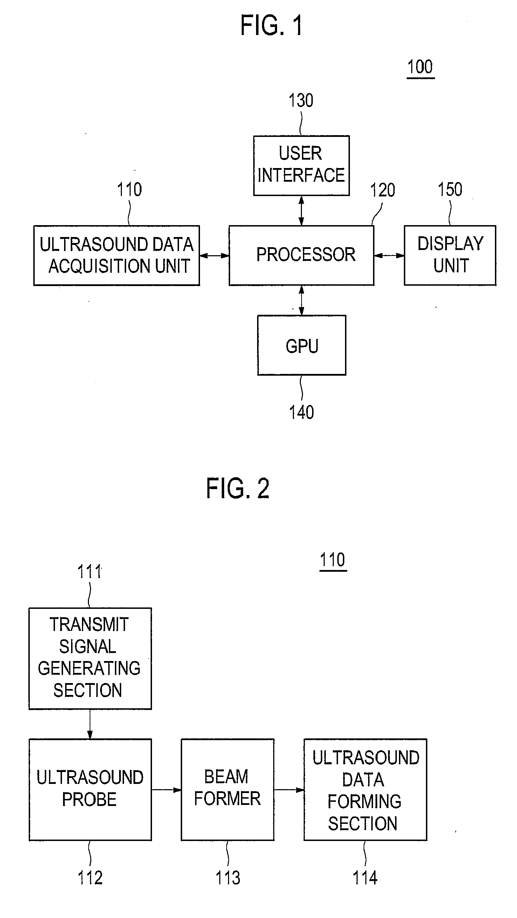

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure. FIG. 1 is a block diagram showing an illustrative embodiment of an ultrasound system. The ultrasound system 100 may include an ultrasound data acquisition unit 110, a processor 120, a user interface 130, a graphic processing unit (GPU) 140 and a display unit 150.

The ultrasound data acquisition unit 110, which is coupled to the processor 120, may transmit ultrasound signals to a target object (not shown) and receive ultrasound echo signals reflected from the target object. The ultrasound data acquisition unit 110 may further form ultrasound data indicative of the target object based on the received ultrasound echo signals.

FIG. 2 is a block diagram showing an illustrative embodiment of the ultrasound data acquisition unit 110. The ultrasound data acquisition unit 110 may include a transmit (Tx) signal generating section 111, an ultrasound probe 112 including a plurality of transducer elements (not shown), a beam former 113 and an ultrasound data forming section 114.

The Tx signal generating section 111 may generate Tx signals according to an image mode set in the ultrasound system 100. The image mode may include a brightness (B) mode, a Doppler (D) mode, a color flow mode, etc. In one exemplary embodiment, the B mode may be set in the ultrasound system 100 to obtain a B mode ultrasound image. The Tx signal generating section 111 may further apply delays to the Tx signals in consideration of distances between the respective transducer elements and focal points. The ultrasound probe 112 may receive the Tx signals from the Tx signal generating section 111 and generate ultrasound signals, which may travel into the target object. The ultrasound probe 112 may further receive ultrasound echo signals reflected from the target object and convert them into electrical receive signals. In such a case, the electrical receive signals may be analog signals. The ultrasound probe 112 may be a three-dimensional probe, a two-dimensional probe, a one-dimensional probe or the like.

The beam former 113 may convert the electrical receive signals outputted from the ultrasound probe 112 into digital signals. The beam former 113 may further apply delays to the digital signals in consideration of the distances between the transducer elements and focal points to thereby output receive-focused signals.

The ultrasound data forming section 114 may form a plurality of ultrasound data by using the receive-focused signals. In one embodiment, the plurality of ultrasound data may be radio frequency (RF) data or IQ data.

Referring back to FIG. 1, the processor 120 may form volume data based on the plurality of ultrasound data transmitted from the ultrasound data forming section 114. The volume data may be comprised of a plurality of frames and include a plurality of voxels each having a brightness intensity.

The user interface 130 may receive a user instruction. The user instruction may include a region of interest (ROI) setting information including a size and a position of the ROI. The user interface 130 may include a control panel (not shown), a mouse (not shown), a keyboard (not shown) or the like.

The GPU 140 coupled to the processor 120 may include a graphic chipset. The GPU 140 may set the ROI on the volume data based on the ROI setting information transmitted from the user interface 130 and render the volume data considering the position of the ROI to thereby form a three-dimensional ultrasound image. Furthermore, the GPU 140 may form a plane image corresponding to the ROI by using the volume data.

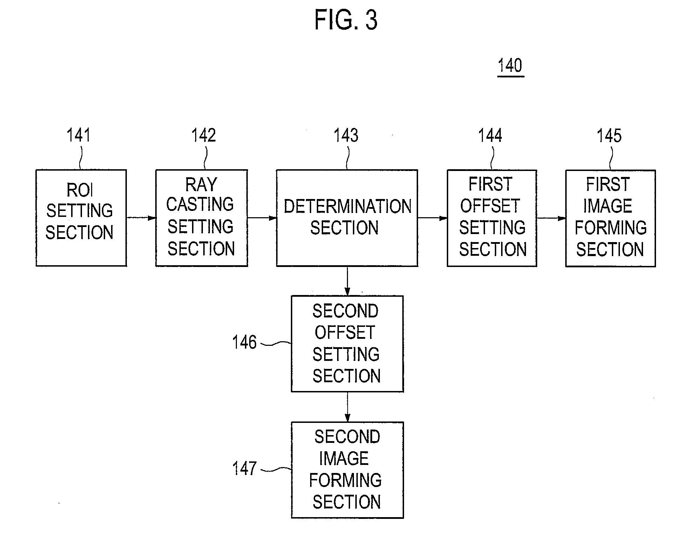

FIG. 3 is a block diagram showing an illustrative embodiment of the GPU 140. The GPU 140 may include a ROI setting section 141, a ray-casting setting section 142, a determination section 143, a first offset setting section 144, a first image forming section 145, a second offset setting section 146 and a second image forming section 147.

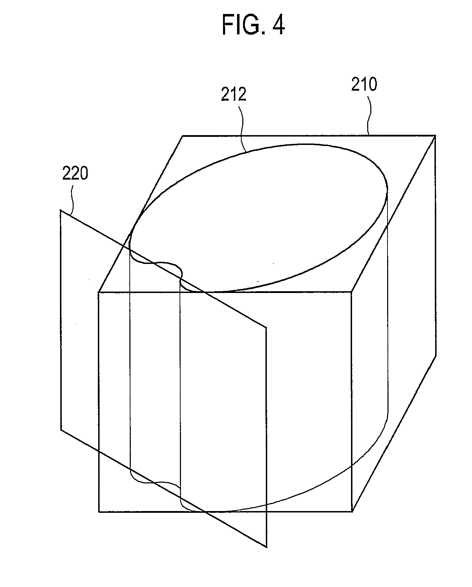

FIG. 4 is a schematic diagram showing an illustrative embodiment of the volume data and the ROI. The ROI setting section 141 may set the ROI 220 on the volume data 210, which may be provided from the processor 120, based on the ROI setting information provided from the user interface 130. For the sake of convenience, although one of planes consisting of the ROI 220 is depicted in FIG. 4, it should be noted herein that the depiction of the ROI is not limited thereto. Reference numeral "212" in FIG. 4 may represent the target object.

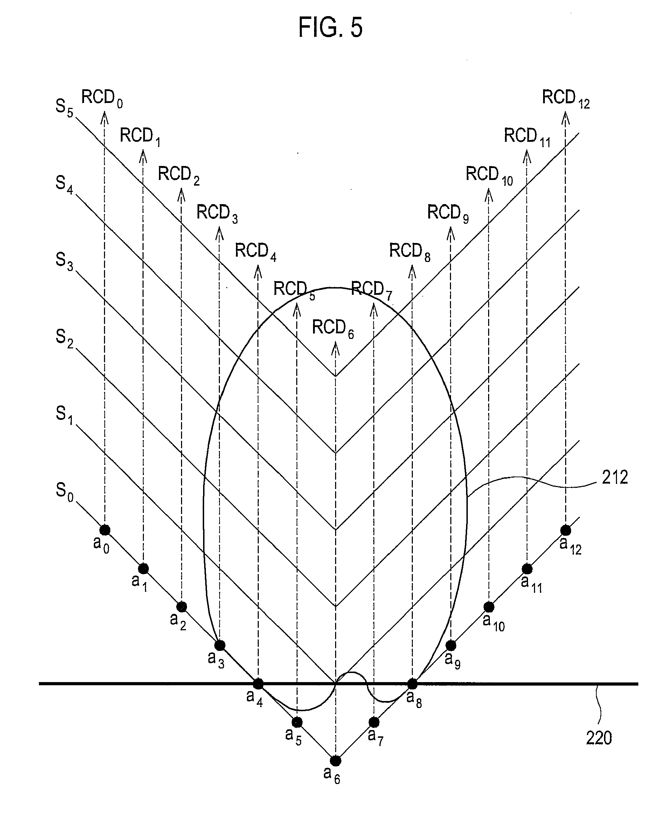

The ray-casting setting section 142 may set a plurality of sampling start points a₀-a₁₂ along edges of the volume data with the ROI set, sampling points S₀-S₅ at a predetermined interval and ray-casting directions RCD₀-RCD₁₂, as illustrated in FIG. 5. For the sake of convenience, although an example of setting six sampling points and thirteen sampling start points are depicted in FIG. 5, it should be noted herein that the setting thereof may not be limited thereto. The sampling start points, the sampling points and the direction of the ray-casting may be set on the volume data by using a variety of well-known methods. Thus, the detailed method of setting the sampling start points, the sampling points and the direction of the ray-casting on the volume data will be not described herein.

The determination section 143 may detect the sampling start points positioned at inside and outside of the ROI 220 to form determination information. The inside of the ROI 220 may represent a side positioned in the ray-casting direction RCD₀-RCD₁₂ from the position of the ROI 220. Further, the outside of the ROI 220 may represent an opposite side of the inside of the ROI 220 based on the position of the ROI 220. In one embodiment, the determination section 143 may detect the sampling start points to determine whether the sampling start points are positioned at the inside or outside of the ROI 220. If the start points are positioned at both the inside and outside of the ROI 220 as shown in FIG. 5, then the determination section 143 may form determination information, which include information on the sampling start points a₀-a₃, a₉-a₁₂ positioned at the inside of the ROI 220 and the sampling start points a₅-a₇ positioned at the outside of the ROI 220.

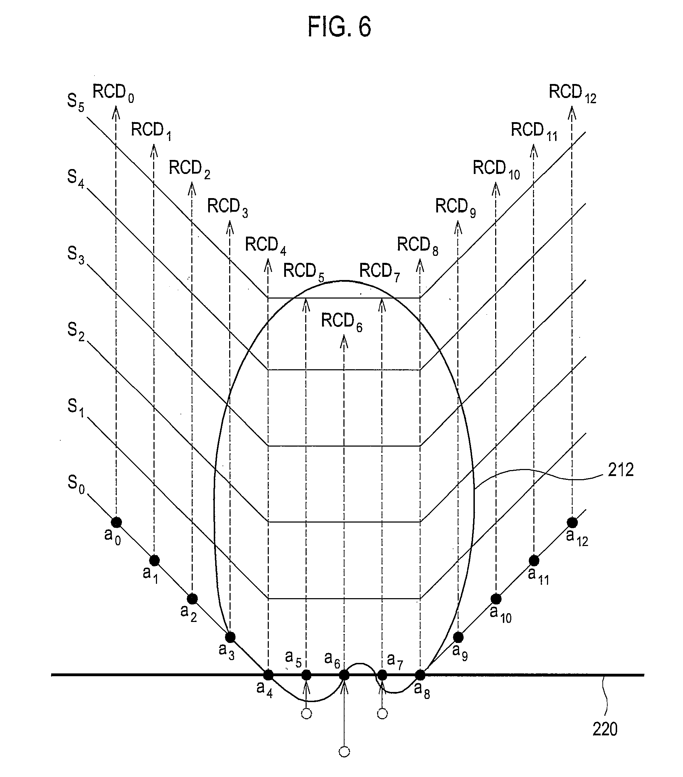

The first offset setting section 144 may set offsets of the sampling start points and the sampling points S₀-S₅ based on the determination information provided from the determination section 143. FIG. 6 is a schematic diagram showing an illustrative embodiment of setting the offsets of the sampling start points. Referring to FIG. 6, if the determination information is provided from the determination section 143, then the first offset setting section 144 may calculate first offsets for the sampling start points a₅-a₇ positioned at the outside of the ROI 220. The first offsets (not shown) may represent the distances between the respective sampling points a₅-a₇ and the ROI 220. The first offset setting section 144 may then move the sampling start points a₅-a₇ positioned at the outside onto the ROI 220 according to the first offsets onto the ROI 220.

The first image forming section 145 may render the volume data from the plurality of sampling start points into the ray-casting direction to thereby form the three-dimensional ultrasound image corresponding to the ROI 220.

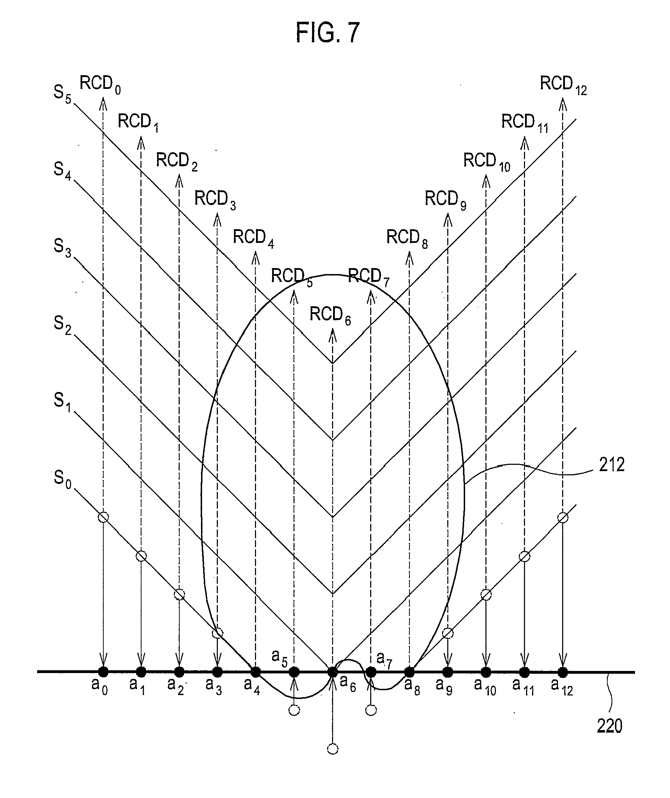

The second offset setting section 146 may set offsets of the sampling start points based on the determination information provided from the determination section 143. FIG. 7 is a schematic diagram showing an illustrative embodiment of setting the sampling start points according to the offsets. Referring to FIG. 7, the second offset setting section 146 may calculate second offsets for both the sampling start points a₅-a₇ positioned at the outside of the ROI 220 and the sampling start points a₀-a₃, a₉-a₁₂ positioned at the inside of the ROI 220. The second offsets (not shown) may represent the distances between the respective sampling points a₀-a₃, a₀-a₇, a₉-a₁₂ and the ROI 220. The second offset setting section 146 may move the sampling start points a₅-a₇ positioned at the outside of the ROI 220 and the sampling start points a₀-a₃, and a₉-a₁₂ positioned at the inside of the ROI 220 onto the ROI 220 according to the second offsets.

In another embodiment, if the sampling start points are positioned only at the inside of the ROI 220 (not shown), the determination section 143 may form determination information on the sampling start points (not shown) positioned at the inside of the ROI 220. If the determination information is provided from the determination section 143, then the second offset setting section 146 may calculate third offsets for the plurality of sampling start points. The third offsets (not shown) may represent the distances between the respective sampling points (not shown) and the ROI 220. The second offset setting section 146 may move the sampling start points (not shown) onto the ROI 220 according to the third offsets.

Referring back to FIG. 7, the second image forming section 147 may form a plane image corresponding to the ROI 220 by using the sampling start points a₀-a₁₂ positioned at the ROI 220.

Referring back to FIG. 1, the display unit 150 may display the three-dimensional ultrasound image and the plane image formed by the GPU 140. The display unit 150 may include a cathode ray tube (CRT) display, a liquid crystal display (LCD), organic light emitting diodes (OLED) display and the like.

Any reference in this specification to "one embodiment," "an embodiment," "example embodiment," "illustrative embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to affect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a first processor configured to form volume data by using a plurality of ultrasound data;
an user interface coupled to the first processor and being configured to receive region of interest (ROI) setting information including a size and a position of the ROI; and
a second processor coupled to the first processor and being configured to set a plurality of sampling start points along edges of the volume data, a plurality of sampling points and a ray-casting direction on the volume data based on the ROI setting information, the GPU being further configured to move sampling start points positioned at an inside or outside of the ROI to be positioned at the ROI to render the volume data.

2. The ultrasound system of Claim 1, wherein the second processor includes a graphic processing unit (GPU).

3. The ultrasound system of Claim 2, wherein the GPU comprises:
a ROI setting section configured to set the ROI on the volume data based on the ROI setting information;
a ray-casting setting section configured to set the plurality of sampling start points along the edges of the volume data with the set ROI, the plurality of sampling points and the direction of the ray-casting on the volume data;
a determination section configured to form a determination information by detecting the sampling start points positioned at an inside or the outside of the ROI;
a first offset setting section configured to set offsets of the sampling start points and the sampling points based on the determination information; and
a first image forming section configured to render the volume data from the plurality of sampling start points into the ray-casting direction based on the setting of offsets to thereby form a three-dimensional ultrasound image.

4. The ultrasound system of Claim 3, wherein the determination section is configured to form the determination information including information of the sampling start points positioned at both the inside and the outside of the ROI when the sampling start points are positioned at both the inside and the outside of the ROI.

5. The ultrasound system of Claim 3, wherein the first offset setting section is further configured to:
calculate first offsets for moving the sampling start points positioned at the inside or outside of the ROI onto the ROI; and
move the sampling start points positioned at the inside or outside of the ROI onto the ROI according to the first offsets.

6. The ultrasound system of Claim 4, wherein the GPU further comprises:
a second offset setting section configured to calculate second offsets for moving the sampling start points positioned at both the inside and the outside of the ROI onto the ROI and move the sampling start points positioned at both the inside and the outside of the ROI onto the ROI; and
a second image forming section configured to form a plane image of the ROI by using the moved sampling start points.

7. A method for volume data rendering of an ultrasound system including a processor, an user interface and a graphic processing unit (GPU), comprising:
a) forming a volume data by using a plurality of ultrasound data;
b) receiving region of interest (ROI) setting information including a size and a position of the ROI;
c) setting the ROI on the volume data based on the ROI setting information;
d) setting a plurality of sampling start points along edges of the volume data, a plurality of sampling points and a ray-casting direction on the volume data based on the ROI setting information; and
e) moving sampling start points positioned at an inside or outside of the ROI to be positioned at the ROI to render the volume data.

8. The method of Claim 7, wherein the sampling start points are positioned at both the inside and outside of the ROI.

9. The method of Claim 7, wherein the step e) comprises:
e1) forming a determination information including information of the sampling start points;
e2) setting offsets between the sampling start points and the ROI based on the determination information;
e3) moving the sampling start points on the ROI based on the offsets; and
e4) rendering the volume data from moved the plurality of sampling start points into the ray-casting direction.

10. The method of Claim 8, wherein the step e) comprises:
e1) forming a determination information including information of the sampling start points;
e2) setting offsets between the sampling start points and the ROI based on the determination information;
e3) moving the sampling start points on the ROI based on the offsets; and
e4) rendering the volume data from the moved plurality of sampling start points into the ray-casting direction.

11. The method of Claim 8, wherein the step e) comprises forming a plane image of the ROI by using the moved sampling start points.

12. A computer readable medium comprising instructions that, when executed by a processor performs a volume data rendering method of an ultrasound system, cause the processor to perform steps comprising:
a) forming a volume data by using a plurality of ultrasound data;
b) receiving region of interest (ROI) setting information including a size and a position of the ROI;
c) setting the ROI on the volume data based on the ROI setting information;
d) setting a plurality of sampling start points along edges of the volume data, a plurality of sampling points and a ray-casting direction on the volume data based on the ROI setting information; and
e) moving sampling start points positioned at an inside or outside of the ROI to be positioned at the ROI to render the volume data.

13. The computer readable medium of Claim 12, wherein the sampling start points are positioned at both the inside and outside of the ROI.

14. The computer readable medium of Claim 13, wherein the step e) comprises:
e1) forming a determination information including information of the sampling start points;
e2) setting offsets between the sampling start points and the ROI based on the determination information; and
e3) moving the sampling start points on the ROI based on the offsets; and
e4) rendering the volume data from the moved plurality of sampling start points into the ray-casting direction.
